# EUROPÄISCHE PATENTANMELDUNG

(11) **EP 4 650 805 A1**
(43) Veröffentlichungstag der Anmeldung: **19.11.2025**
(21) Anmeldenummer: 24176304.4
(22) Anmeldetag: 16.05.2024
(51) Int. Cl.: G01R 33/28, A61B 5/00, G01C 21/20, G01R 33/24

(54) **VERFAHREN UND SYSTEM ZUR BESTIMMUNG EINER ORIENTIERUNG UND EINER POSITION EINES BEWEGLICHEN OBJEKTS RELATIV ZU EINEM B0-FELDMAGNETEN**

(71) Anmelder: Siemens Healthineers AG, 91301 Forchheim (DE)
(72) Erfinder: Sukkau, Johann, 91074 Herzogenaurach (DE)
(74) Vertreter: Siemens Healthineers Patent Attorneys

(57) **Zusammenfassung**

Verfahren zur Bestimmung einer Orientierung und einer Position eines beweglichen Objekts (120) relativ zu einem B0-Feldmagneten (110) eines Magnetresonanztomographen (100) in einer X-Z-Koordinatenebene in einem auf den B0-Feldmagneten (110) ausgerichteten X-Y-Z-Koordinatensystem, wobei das Verfahren zumindest die folgenden Schritte umfasst: Bereitstellen (40) von B0-Referenzdaten des B0-Feldmagneten (110) mit charakteristischen Magnetfeldstärken für eine Vielzahl von X-Y-Z-Koordinaten; Bereitstellen (41) von zumindest drei dreidimensionalen Magnetfeldstärkensensoren (145, 146, 147), die in fester Relativposition am Objekt (120) angeordneten sind; Ermitteln (42) von Positionsdaten für einen jeweiligen Magnetfeldstärkensensor (145, 146, 147) durch Auswertung von Messwertanteilen des jeweiligen Magnetfeldstärkensensors (145, 146, 147), die unabhängig von der jeweiligen Orientierung des beweglichen Objekts (120) in der X-Z-Koordinatenebene sind; Filtern (43) der ermittelten Positionsdaten der Magnetfeldstärkensensoren (145, 146, 147) basierend auf der festen Relativposition der Magnetfeldstärkensensoren (145, 146, 147) und bereitstellen gefilterter Positionsdaten; Bereitstellen (44) eines Orientierungswerts und einer Position des beweglichen Objekts (120) relativ zum B0-Feldmagneten (110) basierend auf den gefilterten Positionsdaten.

## Beschreibung

Die vorliegende Erfindung betrifft ein Verfahren und ein System zur Bestimmung einer Orientierung und einer Position eines beweglichen Objekts relativ zu einem B0-Feldmagneten eines Magnetresonanztomographen.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt.

Üblicherweise basiert die Ortskodierung auf einem X-Y-Z-Koordinatensystem. Dabei ist die Z-Koordinatenachse üblicherweise definiert als eine Symmetrieachse des B0-Feldmagneten durch einen Patiententunnel des B0-Feldmagneten in Vorzugsrichtung des B0-Feldes. Die Z-Koordinatenachse ist bei der üblichen Aufstellung eines Magnetresonanztomographen horizontal ausgerichtet und verläuft zentral durch die Öffnung der Wicklungen des B0-Feldmagneten durch einen Aufnahmebereich des B0-Feldmagneten. Das Aufnahmeobjekt wird üblicherweise parallel zu der Z-Koordinatenachse auf einer Patientenliege in den Patiententunnel gebracht. Gemeinsam mit der Z-Koordinatenachse spannen eine X-Koordinatenachse und eine Y-Koordinatenachse einen Raum auf, wobei vorzugsweise die Koordinatenachsen zueinander orthogonal vorgesehen sind und die X-Koordinatenachse horizontal und die Y-Koordinatenachse vertikal ausgerichtet ist.

Eine mobile Patientenliege wird typischerweise durch eine Person manuell nahe an den B0-Feldmagneten herangefahren, wo die Patientenliege mittels einer automatisierten Greifvorrichtung in eine vorbestimmte Position an den B0-Feldmagneten herangezogen wird. Dabei kann die Patientenliege, und damit auch der Patient, teilweise starken Rüttelbewegungen ausgesetzt werden, da eine nicht exakte Positionierung bzw. Orientierung der Patientenliege durch die Greifvorrichtung korrigiert werden muss.

In diesem Zusammenhang hat sich herausgestellt, dass ein Bedarf besteht, ein Verfahren und ein System bereitzustellen, um eine Orientierung und eine Position eines beweglichen Objekts relativ zu einem B0-Feldmagneten eines Magnetresonanztomographen bereitstellen zu können. Insbesondere besteht ein Bedarf, ein Heranfahren einer Patientenliege an den B0-Feldmagneten vereinfachen zu können.

Es ist daher eine Aufgabe der vorliegenden Erfindung eine Lösung bereitzustellen, mit der eine Orientierung und eine Position eines beweglichen Objekts relativ zu einem B0-Feldmagneten eines Magnetresonanztomographen bereitgestellt werden kann. Insbesondere ist es eine Aufgabe der vorliegenden Erfindung ein Heranfahren einer Patientenliege an den B0-Feldmagneten zu vereinfachen.

Diese und andere Aufgaben, die beim Lesen der folgenden Beschreibung noch genannt werden oder vom Fachmann erkannt werden können, werden durch den Gegenstand der unabhängigen Ansprüche gelöst. Die abhängigen Ansprüche bilden den zentralen Gedanken der vorliegenden Erfindung in besonders vorteilhafter Weise weiter.

Erfindungsgemäß wird ein Verfahren zur Bestimmung einer Orientierung und einer Position eines beweglichen Objekts relativ zu einem B0-Feldmagneten eines Magnetresonanztomographen in einer X-Z-Koordinatenebene in einem auf den B0-Feldmagneten ausgerichteten X-Y-Z-Koordinatensystem vorgeschlagen, wobei das Verfahren zumindest die folgenden Schritte umfasst:
Bereitstellen von BO-Referenzdaten des B0-Feldmagneten mit charakteristischen Magnetfeldstärken für eine Vielzahl von X-Y-Z-Koordinaten;
Bereitstellen von zumindest drei dreidimensionalen Magnetfeldstärkensensoren, die in fester Relativposition am Objekt angeordneten sind;
Ermitteln von Positionsdaten für einen jeweiligen Magnetfeldstärkensensor durch Auswertung von Messwertanteilen des jeweiligen Magnetfeldstärkensensors, die unabhängig von der jeweiligen Orientierung des beweglichen Objekts in der X-Z-Koordinatenebene sind;
Filtern der ermittelten Positionsdaten der Magnetfeldstärkensensoren basierend auf der festen Relativposition der Magnetfeldstärkensensoren und bereitstellen gefilterter Positionsdaten;
Bereitstellen eines Orientierungswerts und einer Position des beweglichen Objekts relativ zum B0-Feldmagneten basierend auf den gefilterten Positionsdaten.

Mit anderen Worten schlägt die vorliegende Erfindung vor, in einem ersten Schritt die Positionsdaten für die jeweiligen Magnetfeldstärkensensoren basierend auf Messwertanteilen der Magnetfeldstärkensensoren zu ermitteln, die unabhängig von einer Orientierung der Magnetfeldstärkensensoren in der X-Z-Koordinatenebene sind.

Die so ermittelten Positionsdaten ergeben grundsätzlich keine eindeutigen Punktkoordinaten, sondern Punktdaten mit einer Vielzahl von möglichen Punktkoordinaten, die auch als sogenannte Punktwolke bezeichnet werden. Die so ermittelten Positionsdaten werden in einem weiteren Schritt anhand der bekannten festen Relativpositionen der Magnetfeldstärkensensoren zueinander gefiltert. Im vorliegenden Kontext ist unter einem Filtern zu verstehen, dass aus den ermittelten Positionsdaten/Punktdaten diejenigen Punkte bzw. Punkkoordinaten ausgewählt werden, die in Übereinstimmung mit den bekannten Relativpositionen der Magnetfeldstärkensensoren gebracht werden können. Diesbezüglich können die ermittelten Positionsdaten anhand unterschiedlicher Bedingungen, die sich aus den bekannten Relativpositionen der Magnetfeldstärkensensoren zueinander ergeben, gefiltert werden. Diese gefilterten Positionsdaten können anschließend herangezogen werden, um einen Orientierungswert, beispielsweise in Form eines Winkels des Objekts relativ zum B0-Feldmagneten, und eine Position des Objekts, beispielsweise in Form einer X-Z Koordinaten, mit ausreichend hoher Genauigkeit ermitteln zu können. Dieser Orientierungswert und die Position des Objekts können anschließend beispielsweise herangezogen werden, um Steuerdaten für eine Navigations- und Bewegungseinrichtung des Objekts zu ermitteln.

Grundsätzlich können vorliegend zwei X-Y-Z-Koordinatensysteme bzw. Bezugssysteme unterschieden werden. Einerseits ein Bezugssystem, das auf den B0-Feldmagneten ausgerichtet ist. In diesem Bezugssystem entspricht die Z-Koordinatenachse einer Symmetrieachse des B0-Feldmagneten in Vorzugsrichtung des B0-Feldes, wobei die Koordinatenachsen vorzugsweise orthogonal zueinander vorgesehen sind und die X-Koordinatenachse vorzugsweise horizontal und die Y-Koordinatenachse vorzugsweise vertikal ausgerichtet ist. Ein solches auf den B0-Feldmagneten ausgerichtetes Koordinatensystem wird in der Praxis auch als sogenanntes "Device Coordinate System" (DCS), bezeichnet.

Ein weiteres X-Y-Z-Koordinatensystem kann auf das Objekt bezogen sein. Ist das Objekt beispielsweise eine Patientenliege, kann der Nullpunkt des X-Y-Z-Koordinatensystems mittig auf der vorderen Kante der Patientenliege vorgesehen sein, die Z-Koordinatenachse beispielsweise entlang der Längsachse der Patientenliege, die X-Koordinatenachse vorzugsweise horizontal und die Y-Koordinatenachse vorzugsweise vertikal ausgerichtet sein, wobei die Koordinatenachse wiederum orthogonal zueinander vorgesehen sind. Ein solches auf eine Patientenliege ausgerichtetes Koordinatensystem wird in der Praxis auch als sogenanntes "Table Coordinate System" (TCS), bezeichnet.

Die BO-Referenzdaten des B0-Feldmagneten werden üblicherweise in Form eines 3D-Gitters bereitgestellt, mit einer typischen Auflösung von 1 mm, 5 mm oder 1...10 mm. Die B0 Referenzdaten umfassen dabei eine eindeutige Zuordnung zwischen einem Punkt im Raum und dem B0-Vektor in diesem Punkt. Die Position eines Punktes im Raum, wie beispielsweise eines festen Punktes der Patientenliege, kann in DCS-Koordinaten mit einem Vektor p = (px, py, pz) angegeben werden, wobei px, py, pz die Koordinaten des Punktes in der jeweiligen Raumrichtung sind, beispielsweise angegeben in mm. Die Richtung/Orientierung einer B0-Feldlinie in der X-Z-Ebene, beispielsweise bezeichnet als Winkel α, kann über die atan2-Funktion wie folgt berechnet werden: α = atan2(h.x, h.z).

Vorzugsweise sind die zumindest drei Magnetfeldstärkensensoren in der X-Z-Koordinatenebene angeordnet, und weisen somit dieselbe Y-Koordinaten auf.

Vorzugsweise weisen in einem auf das Objekt ausgerichteten X-Y-Z-Koordinatensystem, zumindest zwei der zumindest drei Magnetfeldstärkensensoren dieselbe Z-Koordinate auf, wobei es bevorzugt ist, dass zumindest zwei der drei Magnetfeldstärkensensoren zudem dieselbe X-Koordinate aufweisen.

Vorzugsweise umfassen die von der Orientierung des beweglichen Objekts unabhängigen Messwertanteile: den Betrag des B0-Feldvektors (abs(h)), die Y-Feldstärkenkomponenten (h.y), Y-Feldstärkenkomponente normiert auf den Betrag des B0-Feldvektors (h.y/abs(h)) und/oder den Betrag des Vektors in der X-Z-Koordinatenebene (abs(h.x, h.z), womit die Länge des Vektors (h.x, h.z) gemeint ist.

Vorzugsweise umfasst der Schritt des Filterns zumindest folgende Schritte:
Ermitteln von Punktpaaren aus den ermittelten Positionsdaten von zwei Magnetfeldstärkensensoren, bei denen der Abstand der festen Relativposition der zwei Magnetfeldstärkensensoren am Objekt entspricht;
Ermitteln eines Orientierungswinkels für die Punkte eines ermittelten Punktpaars und ermitteln von Punktpaaren, bei denen die Orientierungswinkel für die Punkte des Punktpaars gleich sind;
Ermitteln von Punktpaar-Kombinationen, bei denen der Abstand und die Orientierung der Punktpaar-Kombinationen der festen Relativposition der zumindest drei Magnetfeldstärkensensoren entspricht.

Vorzugsweise ist das bewegliche Objekt eine Patientenliege, wobei die Magnetfeldstärkensensoren vorzugsweise im unteren Bereich an den seitlichen Eckbereichen der Patientenliege angeordnet sind.

Vorzugsweise umfasst das Verfahren weiterhin folgenden Schritt:
Bereitstellen von Steuerdaten basierend auf dem bereitgestellten Orientierungswert und der Position des beweglichen Objekts für eine Navigations- und Bewegungseinrichtung, die eingerichtet ist, das bewegliche Objekt zu einer Zielposition relativ vom B0-Feldmagneten zu bewegen. Vorzugsweise werden die Steuerdaten zyklisch bereitgestellt, wobei vorzugsweise zudem zumindest eine Bewegungstrajektorie des beweglichen Objekts und/oder zumindest ein Richtungs- und/oder Geschwindigkeitsvektor des beweglichen Objekts bei der Bereitstellung der zyklischen Steuerdaten berücksichtigt werden. Beispielsweise kann 50- bis 100-mal pro Sekunde die Position und die Orientierung der Patientenliege bezüglich des B0-Feldmagneten bestimmt werden und beispielsweise einem Navigationskontroller zur Verfügung gestellt werden, sodass dadurch eine automatische Navigation und Bewegung der Patientenliege durchgeführt werden kann insbesondere bis zum Andocken der Patientenliege am B0-Feldmagneten. Vorzugsweise wird die Position der Patientenliege durch einen 2D-Vektor angegeben, mit X- und Z-Koordinaten im DCS-Bezugssystem. Die Orientierung der Patientenliege wird vorzugsweise mit einem Winkel (Yaw) in der horizontalen Ebene zwischen der Z-Koordinatenachse des B0-Feldmagneten und der Längsrichtung der Patientenliege angegeben. Der Navigation- und Bewegungseinrichtung wird vorzugsweise somit eine X-Koordinate, eine Z-Koordinate und der Winkel (Yaw) der Patientenliege etwa 50- bis 100-mal pro Sekunde übermittelt, so dass dadurch die Patientenliege zuverlässig im B0-Streufeld bewegt werden kann.

Vorzugsweise umfasst das Verfahren weiterhin die Schritte:
Bereitstellen zumindest eines Kollisionssensors, der am Objekt angeordnet ist und eingerichtet ist, um einen Bereich, um das Objekt zu erfassen und zu ermitteln, ob sich Objekte in einer geplanten Bewegungspfad befindet, wobei vorzugsweise zumindest drei Kollisionssensoren am Objekt vorgesehen sind, und wobei durch den oder die Kollisionssensoren (160) vorzugsweise ein Bereich mit einem Öffnungswinkel von zumindest 90°, vorzugsweise von zumindest 180° und besonders bevorzugt von zumindest 270° erfasst wird. Möglich ist hier der Einsatz von unterschiedlichen Kollisionssensoren, die beispielsweise einen Bereich vor der Patientenliege, seitlich von der Patientenliege abdecken. Beispielsweise kann hier auch ein LIDAR-Sensor eingesetzt werden.

Wie bereits ausgeführt, ist es bevorzugt, dass der B0-Feldmagnet einen Patiententunnel des Magnetresonanztomographen umschließt, wobei die Z-Koordinatenachse durch eine Symmetrieachse des B0-Feldmagneten in Vorzugsrichtung des B0-Feldes definiert ist, wobei die Koordinatenachsen vorzugsweise orthogonal zueinander vorgesehen sind und die X-Koordinatenachse vorzugsweise horizontal und die Y-Koordinatenachse vorzugsweise vertikal ausgerichtet ist.

Vorzugsweise ist ein Magnetfeldstärkesensor eingerichtet, um eine Feldstärke von drei Komponenten des B0-Feldes in drei Richtungen zu erfassen, die einen Raum aufspannen.

Die vorliegende Erfindung betrifft weiterhin ein System zur Bestimmung einer Orientierung und einer Position eines beweglichen Objekts relativ zu einem B0-Feldmagneten eines Magnetresonanztomographen in einer X-Z-Koordinatenebene in einem auf den B0-Feldmagneten ausgerichteten X-Y-Z-Koordinatensystem, wobei das System umfasst:
eine erste Schnittstelle, eingerichtet zum Empfangen von BO-Referenzdaten des B0-Feldmagneten mit charakteristischen Magnetfeldstärken für eine Vielzahl von X-Y-Z-Koordinaten;
eine zweite Schnittstelle, eingerichtet zum Empfangen von Messwerten der zumindest drei Magnetfeldstärkensensoren;
eine Rechnereinheit, die mit den Schnittstellen verbunden ist und dazu konfiguriert ist, um das oben beschriebene Verfahren auszuführen.

Weiterhin betrifft die vorliegende Erfindung eine Verwendung eines beweglichen Objekts mit zumindest drei dreidimensionalen Magnetfeldstärkensensoren in einem oben beschriebenen Verfahren. Das bewegliche Objekt ist dabei vorzugsweise eine oben beschriebene Patientenliege.

Weiterhin betrifft die vorliegende Erfindung ein Computerprogramm-Element mit Anweisungen, die bei Ausführung auf Datenverarbeitungsgeräten einer Datenverarbeitungsumgebung eingerichtet sind, um die Schritte des oben angeführten Verfahrens in einem oben angeführten System auszuführen.

Alle hierin beschriebenen Ausführungsformen können miteinander kombiniert werden, soweit nicht explizit etwas anderes angegeben ist. Weitere Merkmale, Vorteile und Anwendungsmöglichkeiten der vorliegenden Erfindung ergeben sich aus der nachfolgenden Beschreibung, dem Ausführungsbeispiel und den Figuren. Darin zeigen:
- **Figur 1**: zeigt eine Draufsicht auf ein erfindungsgemäßes System, in Form eines Magnetresonanztomographen;
- **Figur 2**: zeigt eine Seitenansicht des in Figur 1 gezeigten Magnetresonanztomographen;
- **Figur 3**: zeigt eine bevorzugte Anordnung von drei Magnetfeldstärkensensoren relativ zueinander;
- **Figur 4**: zeigt eine schematische Darstellung eines erfindungsgemäßen Verfahrens zur Bestimmung einer Orientierung und einer Position eines beweglichen Objekts relativ zu einem B0-Feldmagneten eines Magnetresonanztomographen in einer X-Z-Koordinatenebene in einem auf den B0-Feldmagneten ausgerichteten X-Y-Z-Koordinatensystem;
- **Figur 5**: zeigt eine schematische Darstellung der Messwertanteile eines Magnetfeldstärkensensors, die unabhängig von seiner Orientierung sind;
- **Figur 6**: zeigt eine schematische Darstellung der Punktwolken zweier Magnetfeldstärkensensoren; und
- **Figur 7**: zeigt eine schematische Darstellung der Punktwolken dreier Magnetfeldstärkensensoren.

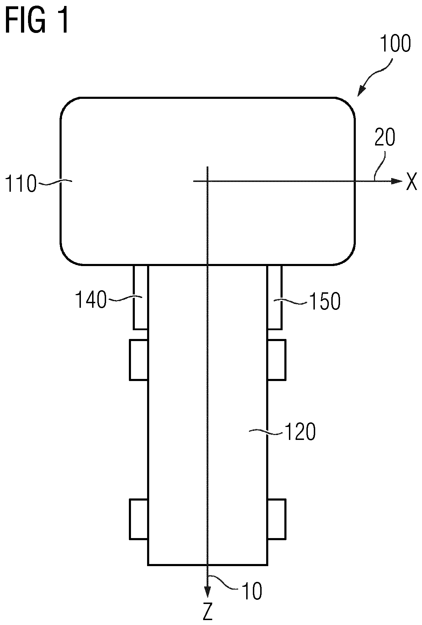

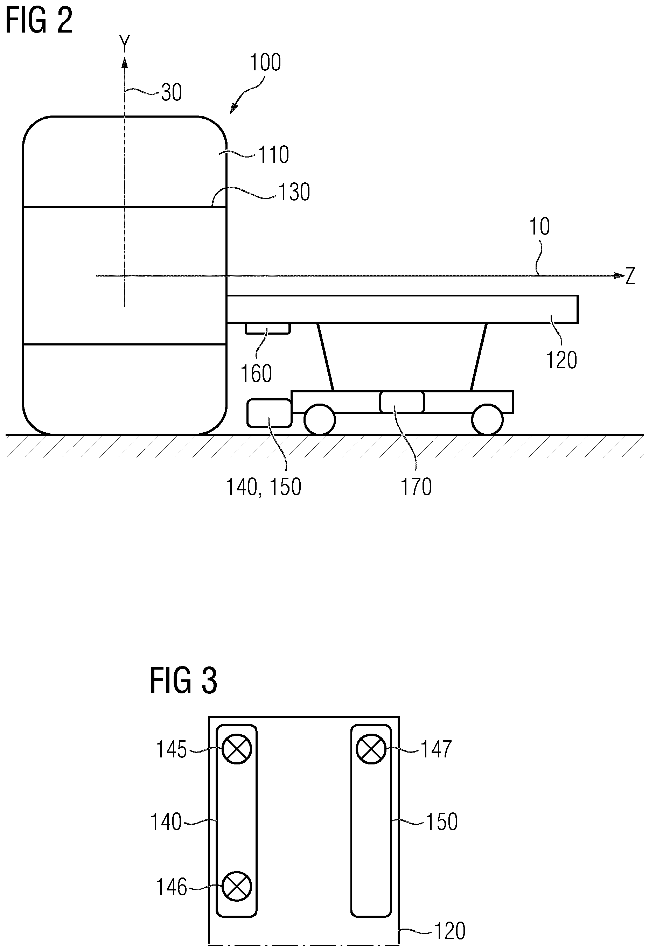

Die **Figur 1** zeigt eine Draufsicht und **Figur 2** eine Seitenansicht auf ein erfindungsgemäßes System 100, in Form eines Magnetresonanztomographen 100. Der Magnetresonanztomographen 100 umfasst insbesondere einen B0-Feldmagenten 110 und eine Patientenliege 120.

Wie in den **Figuren 1** **und** **2** gezeigt, basiert die Ortskodierung auf einem X-Y-Z-Koordinatensystem, das auf den B0-Feldmagneten bezogen ist, ein sogenanntes DCS-Bezugssystem. Dabei ist die Z-Koordinatenachse 10 wie üblich definiert als eine Symmetrieachse des B0-Feldmagneten 110 durch einen Patiententunnel 130 des B0-Feldmagneten 110 in Vorzugsrichtung des B0-Feldes. Die Z-Koordinatenachse 10 ist bei der gezeigten, üblichen Aufstellung des Magnetresonanztomographen 100 horizontal ausgerichtet und verläuft zentral durch die Öffnung der Wicklungen des B0-Feldmagneten 110 durch den Patiententunnel 130 des B0-Feldmagneten 110. Das Aufnahmeobjekt wird üblicherweise parallel zu der Z-Koordinatenachse 10 auf der Patientenliege 120 in den Patiententunnel 130 gebracht. Gemeinsam mit der Z-Koordinatenachse 10 spannen eine X-Koordinatenachse 20 und eine Y-Koordinatenachse 30 einen Raum auf, wobei die X-Y-Z-Koordinatenachsen vorzugsweise orthogonal zueinander vorgesehen sind und wobei die X-Koordinatenachse horizontal und die Y-Koordinatenachse vertikal ausgerichtet ist. Die Patientenliege 120 umfasst weiterhin eine erste Magnetfeldstärkensensoreinheit 140, eine zweite Magnetfeldstärkensensoreinheit 150, eine Kollisionssensoreinheit 160 und eine Auswerte- und Navigationseinheit 170. Wie in **Figur 3** gezeigt, umfasst die erste Magnetfeldstärkensensoreinheit 140 im gezeigten Ausführungsbeispiel zwei dreidimensionale Magnetfeldstärkensensoren 145, 146 und die zweite Magnetfeldstärkensensoreinheit 150 eine Magnetfeldstärkensensoren 147. Die Abstände und Positionierung der Magnetfeldstärkensensoren 145, 146, 147 relativ zueinander sind dabei bekannt.

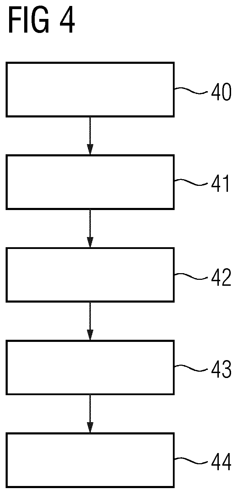

**Figur 4** zeigt eine schematische Darstellung eines erfindungsgemäßen Verfahrens zur Bestimmung der Orientierung und der Position des beweglichen Objekts 120 relativ zum B0-Feldmagneten 110 des Magnetresonanztomographen 100 in der X-Z-Koordinatenebene im DCS-Bezugssystem.

In einem ersten Schritt 40 werden BO-Referenzdaten des B0-Feldmagneten 110 mit charakteristischen Magnetfeldstärken für eine Vielzahl von X-Y-Z-Koordinaten bereitgestellt. In einem weiteren Schritt 41 werden zumindest drei dreidimensionale Magnetfeldstärkensensoren 145, 146, 147, die in fester Relativposition am Objekt 120 angeordneten sind, bereitgestellt. In einem weiteren Schritt 42 werden Positionsdaten für die jeweiligen Magnetfeldstärkensensoren 145, 146, 147 durch Auswertung von Messwertanteilen des jeweiligen Magnetfeldstärkensensors 145, 146, 147 bereitgestellt, die unabhängig von der jeweiligen Orientierung des beweglichen Objekts 120 in der X-Z-Koordinatenebene sind. In einem weiteren Schritt 43 werden die ermittelten Positionsdaten der Magnetfeldstärkensensoren 145, 146, 147 basierend auf der festen Relativposition der Magnetfeldstärkensensoren 145, 146, 147 und Bereitstellen gefilterter Positionsdaten ermittelt. Schließlich werden in einem Schritt 44 ein Orientierungswert und eine Position des beweglichen Objekts 120 relativ zum B0-Feldmagneten 110 basierend auf den gefilterten Positionsdaten bereitgestellt.

Nachfolgend wird eine besonders bevorzugte Ausgestaltung eines erfindungsgemäßen Verfahrens und Systems beschrieben. In der bevorzugten Ausgestaltung des erfindungsgemäßen Verfahrens wird der Orientierungswert und die Position des beweglichen Objekts 120 zur Bereitstellung von Steuerdaten für eine Navigations- und Bewegungseinrichtung des Objekts 120, hier in Form einer autonom fahrbaren Patientenliege 120, genutzt.

Die Patientenliege 120 umfasst in der bevorzugten Ausgestaltung zumindest drei dreidimensionale Magnetfeldstärkensensoren 145, 146, 147. Die Messdaten der Magnetfeldstärkensensoren 145, 146, 147 können an die Auswerte- und Navigationseinheit 170 übermittelt werden und durch diese in Steuerdaten verarbeitet werden. In der Auswerte- und Navigationseinheit 170 können die Messdaten zusammengefasst werden und durch Anwendung des erfindungsgemäßen Verfahrens können Orientierungswerte und die Position der Patientenliege 120 relativ zum B0-Feldmagneten 110 bereitgestellt werden. Vorzugsweise werden dabei eine X-Koordinate, eine Z-Koordinate und der Winkel (Yaw) der Patientenliege 120 etwa 50- bis 100-mal pro Sekunde ermittelt. Die Auswerte- und Navigationseinheit 170 kann daraufhin den nächsten Wegpunkt auf dem Weg zum Andocken der Patientenliege 120 am B0-Feldmagneten 110 ermitteln und beispielsweise entsprechende Steuerbefehle an Servomotoren zum Bewegen und Lenken der Patientenliege 120 übermitteln. Zusätzlich können Daten der Kollisionssensoreinheit 160 an die Auswerte- und Navigationseinheit 170 übermittelt werden, um sicherzustellen, dass sich keine Objekte/Personen im Fahrtweg der Patientenliege 120 befinden.

Vorzugsweise sind die Magnetfeldstärkensensoreinheiten 140, 150, und damit die darin angeordneten Magnetfeldstärkensensoren 145, 146, 147, im unteren Bereich an der Patientenliege 120, möglichst in Bodennähe angeordnet. Die Magnetfeldstärkensensoreinheiten 140, 150 sind dabei vorzugsweise seitlich an der Patientenliege 120 angeordnet. Damit kann erreicht werden, dass das B0-Magnetfeld, dass die Magnetfeldstärkensensoren 145, 146, 147 messen auch in der direkten Näher des B0-Feldmagneten 110 vergleichsweise klein ist, sodass das B0 Magnetfeld auch mit kostengünstigen Magnetfeldstärkensensoren gemessen werden kann. Darüber hinaus kann durch die bevorzugte Anordnung der Magnetfeldstärkensensoreinheiten 140, 150 erreicht werden, dass in der Nähe der Magnetfeldstärkensensoren 145, 146, 147 keine großen Metallteile der Patientenliege 120 vorgesehen sind, und dass B0-Magnetfeld, das durch die Magnetfeldstärkensensoren 145, 146, 147 gemessen wird, nicht gestört wird.

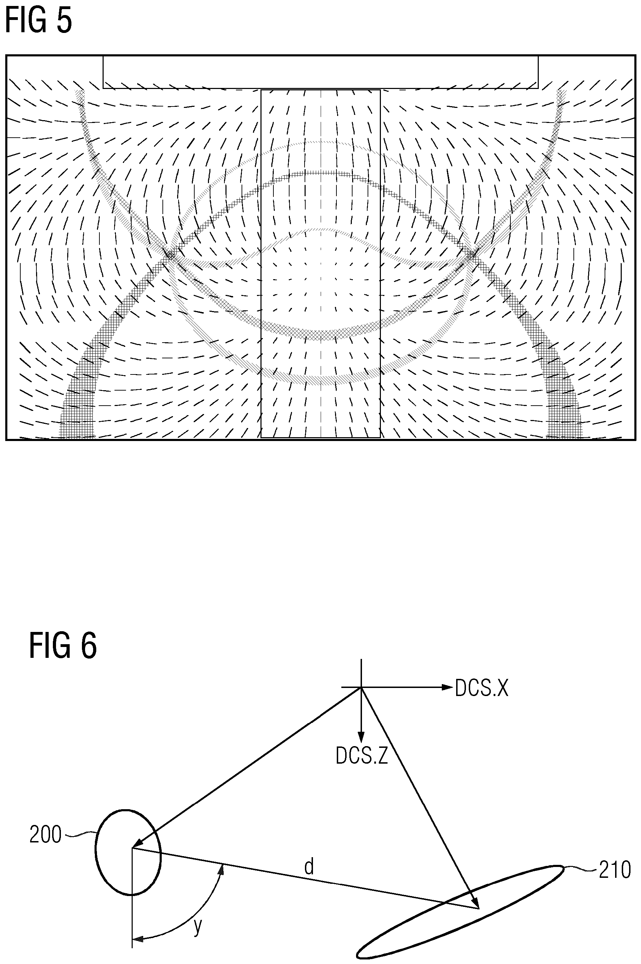

Wie ausgeführt, werden zunächst die Positionsdaten für die Magnetfeldstärkensensoren 145, 146, 147 durch Auswertung von Messwertanteilen des jeweiligen Magnetfeldstärkensensors 145, 146, 147, die unabhängig von der jeweiligen Orientierung des beweglichen Objekts in der X-Z-Koordinatenebene sind, ermittelt. Die von der Orientierung eines Magnetfeldstärkensensors 145, 146, 147 abhängigen Werte h.x und h.z werden hier nicht verwendet. In **Figur 5** sind die jeweiligen Messwertanteile dargestellt. Die Schnittmenge der Messwerteanteile stellen die Positionsdaten/Punktwolken eines Magnetfeldstärkensensors 145, 146, 147 dar. Grundsätzlich ist die Punktwolke umso größer, je weiter der Magnetfeldstärkensensor 145, 146, 147 vom B0-Feldmagneten 110 entfernt ist. Je weiter sich der Magnetfeldstärkensensor 145, 146, 147 dem B0-Feldmagneten 110 nähert, umso kleiner wird die Punktwolke. Diese zerfällt aufgrund der Rotationssymmetrie des B0-Feldes um die Z-Koordinatenachse in zwei oder sogar vier Teile.

In einem weiteren Schritt werden die Punktwolken 200, 210 von zwei der Magnetfeldstärkensensoren 145, 146, 147 miteinander kombiniert. Wie in **Figur 6** illustriert, werden dabei diejenigen Punkte-Paare ermittelt/herausgefiltert, bei denen der Abstand d dem tatsächlichen Abstand der betrachteten zwei Magnetfeldstärkensensoren 145, 146, 147 entspricht. Entsprechende Punkte-Paare werden für die unterschiedlichen Zweier-Kombinationen der Magnetfeldstärkensensoren 145, 146, 147 ermittelt. Hierbei ist zu berücksichtigen, dass vorzugsweise Punkte-Paare ermittelt werden, die dem bekannten Abstand d innerhalb einer vorgegebenen Toleranz entsprechen. Beispielsweise können Punkte-Paare mit einer Toleranz von +/-3%, +/-2% oder +/-1% vom jeweiligen Wert des Messanteils gebildet werden. Alternativ können andere Toleranzgrenzen/-bereiche herangezogen werden.

In einem weiteren Schritt werden die oben ermittelten Punkte-Paare zweier Magnetfeldstärkensensoren 145, 146, 147 hinsichtlich ihrer Orientierung gefiltert. Denn aufgrund der festen Installation der Magnetfeldstärkensensoren 145, 146, 147 an der Patientenliege 120 weisen alle Magnetfeldstärkensensoren 145, 146, 147 die gleiche Orientierung, d.h. den gleichen Yaw-Winkel, auf. Die Orientierung, dargestellt in Form des Yaw-Winkels, kann zu jedem ermittelten Punkte-Paar berechnet werden. Dies kann beispielsweise mittels der atan2-Funktion erfolgen, wobei α = atan2(h.x, h.z) ist. Aus den oben ermittelten Punkte-Paaren zweier Magnetfeldstärkensensoren 145, 146, 147 werden daher diejenigen Punkte-Paare ermittelt/herausgefiltert, bei denen die Orientierung der Magnetfeldstärkensensoren 145, 146, 147 gleich ist. Auch hier ist es bevorzugt, dass Punkte-Paare ermittelt werden, die eine gleiche Orientierung innerhalb einer vorgegebenen Toleranz aufweisen. Beispielsweise können Punkt-Paare gefiltert werden, die mit einer Toleranz von +/-3%, +/-2% oder +/-1% eine gleiche Orientierung aufweisen. Alternativ können auch hier andere Toleranzgrenzen/-bereiche herangezogen werden.

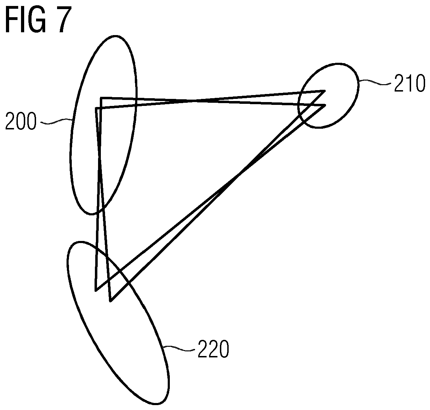

In einem weiteren Schritt werden die ermittelten Punkte-Paare der Magnetfeldstärkensensoren 145, 146, 147 mit gleicher Orientierung nochmals gefiltert, in dem diejenigen Punkte-Paare der Magnetfeldstärkensensoren 145, 146, 147 ermittelt werden, die in Kombination der tatsächlichen Geometrie der zumindest drei Magnetfeldstärkensensoren 145, 146, 147 entsprechen. In **Figur 7** sind beispielhaft zwei Lösungen dieser Punkte-Paare Kombinationen gezeigt, die die tatsächliche Geometrie der zumindest drei Magnetfeldstärkensensoren 145, 146, 147 abbilden. Im bevorzugten Ausführungsbeispiel sind somit diejenigen Punkte-Paare der Magnetfeldstärkensensoren 145, 146, 147 zu ermitteln, die der in **Figur 3** gezeigten dreiecksförmigen Anordnung der drei Magnetfeldstärkensensoren 145, 146, 147 entsprechen. Auch hier ist es bevorzugt, entsprechende Toleranzen vorzugeben. Beispielsweise können auch hier Kombinationen gefiltert werden, die mit einer Toleranz von +/-3%, +/-2% oder +/-1% die tatsächliche Geometrie der zumindest drei Magnetfeldstärkensensoren 145, 146, 147 entsprechen. Alternativ können auch hier andere Toleranzgrenzen/-bereiche herangezogen werden.

Die verbleibenden Punkte-Paare der Magnetfeldstärkensensoren 145, 146, 147 werden zur Ermittlung einer Position eines Magnetfeldstärkensensors 145, 146, 147 herangezogen. Diese Positionen der Magnetfeldstärkensensoren 145, 146, 147 werden anschließend verwendet, um jeweils eine X-Koordinate, eine Z-Koordinate und einen Winkelwert der Patientenliege 120 zu ermitteln. Ein jeweiliger Mittelwert der so ermittelten X-Koordinaten, Z-Koordinaten und Winkelwerte kann schließlich zur Bereitstellung der Steuerdaten für die Navigations- und Bewegungseinrichtung der Patientenliege 120 herangezogen werden, um die Patientenliege 120 zu einer Zielposition relativ zum B0-Feldmagneten 110 zu bewegen. Vorzugsweise werden die Steuerdaten zyklisch bereitgestellt, wobei vorzugsweise zudem zumindest eine Bewegungstrajektorie der Patientenliege 120 und/oder zumindest ein Richtungs- und/oder Geschwindigkeitsvektor der Patientenliege 120 bei der Bereitstellung der zyklischen Steuerdaten berücksichtigt werden. Beispielsweise kann 50- bis 100-mal pro Sekunde die Position und die Orientierung der Patientenliege 120 bezüglich des B0-Feldmagneten 110 bestimmt werden und beispielsweise einem Navigationskontroller zur Verfügung gestellt werden, sodass dadurch eine automatische Navigation und Bewegung der Patientenliege 120 durchgeführt werden kann, insbesondere bis zum Andocken der Patientenliege 120 am B0-Feldmagneten 110.

Vorzugsweise wird die Position der Patientenliege 120 durch einen 2D-Vektor angegeben, mit X- und Z-Koordinaten im DCS-Bezugssystem. Die Orientierung der Patientenliege wird vorzugsweise mit einem Winkel (Yaw) in der horizontalen Ebene zwischen der Z-Koordinatenachse des B0-Feldmagneten 110 und der Längsrichtung der Patientenliege 120 angegeben. Der Navigation- und Bewegungseinrichtung wird vorzugsweise somit eine X-Koordinate, eine Z-Koordinate und der Winkel (Yaw) der Patientenliege 120 etwa 50- bis 100-mal pro Sekunde übermittelt, so dass dadurch die Patientenliege 120 zuverlässig im B0-Magnetfeld bewegt werden kann.

Im Ergebnis kann durch das erfindungsgemäße Verfahren bzw. durch das erfindungsgemäße System eine Patientenliege 120 automatisch und sanft an einen B0-Feldmagneten 110 angedockt werden, was den Patientenkomfort deutlich erhöht. Das automatische Andocken kann zudem vergleichsweise schnell erfolgen, insbesondere im Vergleich zu einem manuellen Bewegen der Patientenliege 120 durch eine Bedienperson. Das erfindungsgemäße Verfahren bzw. das erfindungsgemäße System kann selbstständig auch für andere Anwendungen eingesetzt werden, wenn eine Position und eine Orientierung eines Objekts relativ zu einem B0-Feldmagneten zuverlässig, genau und schnell bereitgestellt werden sollen.

Die vorliegende Erfindung ist nicht auf die vorhergehend beschriebene Ausführungsform beschränkt, solange sie vom Gegenstand der nachfolgenden Ansprüche umfasst ist. Insbesondere ist die vorliegende Erfindung nicht auf die Verwendung der drei Magnetfeldstärkensensoren 145, 146, 147 beschränkt. Durch den Einsatz weiterer Magnetfeldstärkensensoren können vorteilhafte Redundanzen, eine höhere Genauigkeit und Zuverlässigkeit sowie eine Immunität gegen Messrauschen bereitgestellt werden. Vorzugsweise umfassen die Magnetfeldstärkensensoreinheiten jeweils bis zu 12 Magnetfeldstärkensensoren, sodass bei Einsatz von drei Magnetfeldstärkensensoreinheiten insgesamt 36 Magnetfeldstärkensensoren eingesetzt werden können.

Ergänzend wird darauf hingewiesen, dass die Begriffe "umfassend" und "aufweisend" keine anderen Elemente oder Schritte ausschließen und die unbestimmten Artikel "eine" oder "ein" keine Vielzahl ausschließen. Ferner wird darauf hingewiesen, dass Merkmale oder Schritte, die mit Verweis auf obige Ausführungsformen beschrieben worden sind, auch in Kombination mit anderen Merkmalen verwendet werden können.

Darüber hinaus wird darauf hingewiesen, dass unabhängig vom grammatikalischen Geschlecht eines bestimmten Begriffs, Personen mit männlicher, weiblicher oder anderer Geschlechteridentität mit umfasst sind.

## Patentansprüche

1. Verfahren zur Bestimmung einer Orientierung und einer Position eines beweglichen Objekts (120) relativ zu einem B0-Feldmagneten (110) eines Magnetresonanztomographen (100) in einer X-Z-Koordinatenebene in einem auf den B0-Feldmagneten (110) ausgerichteten X-Y-Z-Koordinatensystem, wobei das Verfahren zumindest die folgenden Schritte umfasst:
Bereitstellen (40) von BO-Referenzdaten des B0-Feldmagneten (110) mit charakteristischen Magnetfeldstärken für eine Vielzahl von X-Y-Z-Koordinaten;
Bereitstellen (41) von zumindest drei dreidimensionalen Magnetfeldstärkensensoren (145, 146, 147), die in fester Relativposition am Objekt (120) angeordneten sind;
Ermitteln (42) von Positionsdaten für einen jeweiligen Magnetfeldstärkensensor (145, 146, 147) durch Auswertung von Messwertanteilen des jeweiligen Magnetfeldstärkensensors (145, 146, 147), die unabhängig von der jeweiligen Orientierung des beweglichen Objekts (120) in der X-Z-Koordinatenebene sind;
Filtern (43) der ermittelten Positionsdaten der Magnetfeldstärkensensoren (145, 146, 147) basierend auf der festen Relativposition der Magnetfeldstärkensensoren (145, 146, 147) und bereitstellen gefilterter Positionsdaten;
Bereitstellen (44) eines Orientierungswerts und einer Position des beweglichen Objekts (120) relativ zum B0-Feldmagneten (110) basierend auf den gefilterten Positionsdaten.

2. Verfahren nach Anspruch 1, wobei die zumindest drei Magnetfeldstärkensensoren (145, 146, 147) in der X-Z-Koordinatenebene angeordnet sind, und somit dieselbe Y-Koordinaten aufweisen.

3. Verfahren nach Anspruch 1 oder Anspruch 2, wobei in einem auf das bewegliche Objekt (120) ausgerichteten X-Y-Z-Koordinatensystem, zumindest zwei der zumindest drei Magnetfeldstärkensensoren (145, 146, 147) dieselbe Z-Koordinate aufweisen und zumindest zwei der drei Magnetfeldstärkensensoren (145, 146, 147) dieselbe X-Koordinate aufweisen.

4. Verfahren nach einem der vorhergehenden Ansprüche, wobei die von der Orientierung des beweglichen Objekts (120) unabhängigen Messwertanteile umfassen: den Betrag des B0-Feldvektors (abs(h)), die Y-Feldstärkenkomponenten (h.y), Y-Feldstärkenkomponente normiert auf den Betrag des B0-Feldvektors (h.y/abs(h)) und/oder den Betrag des Vektors in der X-Z-Koordinatenebene (abs(h.x, h.z).

5. Verfahren nach einem der vorhergehenden Ansprüche, wobei die Positionsdaten als Punktdaten/Punktwolken bereitgestellt werden.

6. Verfahren nach einem der vorhergehenden Ansprüche, wobei der Schritt des Filterns umfasst:
Ermitteln von Punktpaaren aus den ermittelten Positionsdaten von zwei Magnetfeldstärkensensoren (145, 146, 147), bei denen der Abstand (d) der festen Relativposition der zwei Magnetfeldstärkensensoren (145, 146, 147) am Objekt (120) entspricht;
Ermitteln eines Orientierungswinkels für die Punkte eines ermittelten Punktpaars und ermitteln von Punktpaaren, bei denen die Orientierungswinkel für die Punkte des Punktpaars gleich sind;
Ermitteln von Punktpaar-Kombinationen, bei denen der Abstand und die Orientierung der Punktpaar-Kombinationen der festen Relativposition der zumindest drei Magnetfeldstärkensensoren (145, 146, 147) entspricht.

7. Verfahren nach einem der vorhergehenden Ansprüche, wobei das bewegliche Objekt (120) eine Patientenliege (120) ist, und wobei die Magnetfeldstärkensensoren (145, 146, 147) im unteren Bereich an den seitlichen Eckbereichen der Patientenliege (120) angeordnet sind.

8. Verfahren nach einem der vorhergehenden Ansprüche, weiterhin umfassend:
Bereitstellen von Steuerdaten basierend auf dem bereitgestellten Orientierungswert und der Position des beweglichen Objekts (120) für eine Navigations- und Bewegungseinrichtung (170), die eingerichtet ist, das bewegliche Objekt (120) zu einer Zielposition relativ vom B0-Feldmagneten (110) zu bewegen.

9. Verfahren nach einem der vorhergehenden Ansprüche, wobei die Steuerdaten zyklisch bereitgestellt werden und zudem zumindest eine Bewegungstrajektorie des beweglichen Objekts (120) und/oder zumindest ein Richtungs- und/oder Geschwindigkeitsvektor des beweglichen Objekts (120) bei der Bereitstellung der zyklischen Steuerdaten berücksichtigt werden.

10. Verfahren nach Anspruch 8 oder Anspruch 9, weiterhin umfassend:
Bereitstellen zumindest eines Kollisionssensors (160), der am Objekt (120) angeordnet ist und eingerichtet ist, um einen Bereich, um das Objekt (120) zu erfassen und zu ermitteln, ob sich Objekte in einem geplanten Bewegungspfad befindet, wobei vorzugsweise zumindest drei Kollisionssensoren (160) am Objekt (120) vorgesehen sind, und wobei durch den oder die Kollisionssensoren (160) vorzugsweise ein Bereich mit einem Öffnungswinkel von zumindest 90°, vorzugsweise von zumindest 180° und besonders bevorzugt von zumindest 270° erfasst wird.

11. Verfahren nach einem der vorhergehenden Ansprüche, wobei der B0-Feldmagnet (110) einen Patiententunnel (130) des Magnetresonanztomographen (100) umschließt, wobei die Z-Koordinatenachse (10) durch eine Symmetrieachse des B0-Feldmagneten (110) in Vorzugsrichtung des B0-Feldes definiert ist, wobei die Koordinatenachsen vorzugsweise orthogonal zueinander vorgesehen sind und die X-Koordinatenachse (20) vorzugsweise horizontal und die Y-Koordinatenachse (30) vorzugsweise vertikal ausgerichtet ist.

12. Verfahren nach einem der vorhergehenden Ansprüche, wobei die Magnetfeldstärkesensoren (145, 146, 147) eingerichtet sind, um eine Feldstärke von drei Komponenten (B0.x-,B0.y-,B0.z) des B0-Feldes in drei Richtungen zu erfassen, die einen Raum aufspannen.

13. System zur Bestimmung einer Orientierung und einer Position eines beweglichen Objekts (120) relativ zu einem B0-Feldmagneten (110) eines Magnetresonanztomographen (100) in einer X-Z-Koordinatenebene in einem auf den B0-Feldmagneten (110) ausgerichteten X-Y-Z-Koordinatensystem, wobei das System umfasst:
eine erste Schnittstelle, eingerichtet zum Empfangen von BO-Referenzdaten des B0-Feldmagneten (110) mit charakteristischen Magnetfeldstärken für eine Vielzahl von X-Y-Z-Koordinaten;
eine zweite Schnittstelle, eingerichtet zum Empfangen von Messwerten der zumindest drei Magnetfeldstärkensensoren (145, 146, 147);
eine Rechnereinheit, die mit den Schnittstellen verbunden ist und dazu konfiguriert ist, das Verfahren nach einem der Ansprüche 1 bis 12 auszuführen.

14. Verwendung eines beweglichen Objekts (120) mit zumindest drei dreidimensionalen Magnetfeldstärkensensoren (145, 146, 147) in einem Verfahren nach einem der Ansprüche 1 bis 12, wobei das bewegliche Objekt (120) vorzugsweise eine Patientenliege (120) ist.

15. Computerprogramm-Element mit Anweisungen, die bei Ausführung auf Datenverarbeitungsgeräten einer Datenverarbeitungsumgebung eingerichtet sind, um die Schritte des Verfahrens nach einem der Ansprüche 1 bis 12 in einem System nach Anspruch 13 auszuführen.
